# EUROPEAN PATENT APPLICATION

(11) **EP 1 142 864 A1**
(43) Date of publication of application: **10.10.2001**
(21) Application number: 99961308.6
(22) Date of filing: 22.12.1999
(51) Int. Cl.: C07C 213/02, C07C 215/28

(54) **PROCESS FOR PRODUCING L-ERYTHRO-(1R,2S)-2-AMINO-1-PHENYLPROPAN-1-OL**

(30) Priority: 24.12.1998 JP 36733998
(71) Applicant: NAGASE & COMPANY, LTD., Osaka-shi, Osaka 550-0013 (JP)
(72) Inventor: IKUNAKA, Masaya, Research & Development, Nishi-ku, Kobe-shi, Hyogo 651-2241 (JP); CHIKUSA, Yasuo, Research & Development, Nishi-ku, Kobe-shi, Hyogo 651-2241 (JP); MATSUMOTO, Jun, Research & Development, Nishi-ku, Kobe-shi, Hyogo 651-2241 (JP); HIRAYAMA, Yoshihiko, Research & Development, Nishi-ku, Kobe-shi, Hyogo 651-2241 (JP)
(74) Representative: VOSSIUS & PARTNER
(86) International application number: JP9907206
(87) International publication number: WO0039071

(57) **Abstract**

An efficient process for stereoselectively producing L-erythro-(1R,2S)-2-amino-1-phenylpropan-1-ol from L-(R)-phenylacetylcarbinol, which comprises reductively aminating L-(R)-phenylacetylcarbinol with a primary aralkylamine under catalytic reduction conditions and successively subjecting the resultant L-erythro-(1R,2S)-2-(N-aralkylamino)-1-phenylpropan-1-ol to catalytic reduction to remove the N-aralkyl group in a manner as in hydrogenolysis.

## Description

### TECHNICAL FIELD

The present invention relates to a process for stereoselectively preparing L-erythro-(1R,25)-2-amino-1-phenylpropan-1-ol from L-(R)-phenylacetylcarbinol.

### BACKGROUND ART

L-erythro-(1R,2S)-2-amino-1-phenylpropan-1-ol is used as an optical resolving agent or an asymmetric auxiliary for asymmetric synthesis.

The prior processes for preparing the L-erythro-(1R,2S)-2-amino-1-phenylpropan-1-ol include a process by optical resolution (JP-A 51/98231). However, the process is not efficient because the yield in optical resolution does not exceed 50%.

Also, a process of reductively aminating L-(R)-phenylacetylcarbinol with ammonia as well as a process of catalytically reducing an oxime prepared therefrom are reported in the past (GB Patent No. 365,535). However, neither of the processes is necessarily practical in respect of reaction conditions, yields and stereoselectivity.

### DISCLOSURE OF THE INVENTION

It is an object of the present invention to provide a process for stereoselectively preparing L-erythro-(1R,2S)-2-amino-1-phenylpropan-1-ol using L-erythro-(R)-phenylacetylcarbinol as a starting material.

The present inventors found, as a consequence of an intensive investigation, a process in which L-(R)-phenylacetylcarbinol of formula (I): 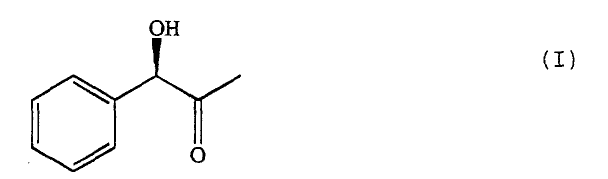 is smoothly reductively-aminated, under catalytic reduction conditions, with a primary aralkylamine of formula (II):

ArCH₂NH₂ (II)

wherein Ar is an aryl group, and subsequently,
the N-aralkyl group in the resultant L-erythro-(1R,2S)-2-(N-aralkylamino)-1-phenylpropan-1-ol of formula (III): 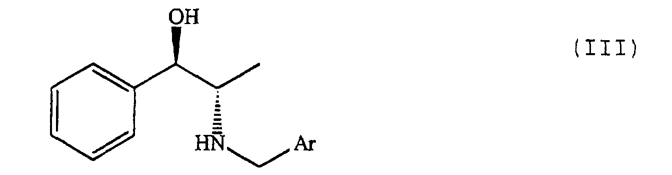 is catalytically hydrogenolyzed to stereoselectively obtain L-erythro-(1R,2S)-2-amino-1-phenylpropan-1-ol of formula (IV):

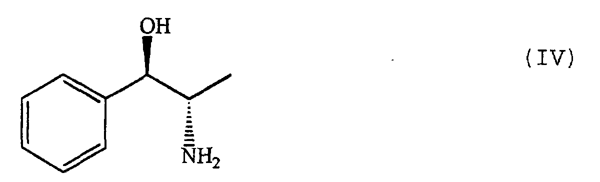

Accordingly, the present invention provides a process for preparing L-erythro-(1R,2S)-2-amino-1-phenylpropan-1-ol of formula (IV), characterized in that L-(R)-phenylacetylcarbinol of formula (I) is reductively aminated with a primary aralkylamine of formula (II) under catalytic reduction conditions, and subsequently, the resultant L-erythro-(1R,2S)-2-(N-aralkylamino)-1-phenylpropan-1-ol of formula (III) is catalytically reduced to remove the N-aralkyl group hydrogenolytically.

### BEST MODE FOR PRACTICING THE INVENTION

According to the present process, the compound of formula (III) can be obtained by reacting the compound of formula (I) with the compound of formula (II) under hydrogen atmosphere in a suitable solvent in the presence of a catalyst for catalytic reduction.

L-(R)-Phenylacetylcarbinol of formula (I) is a known substance and can be prepared, for example, according to the method by D.H.G. Crout et al., J. Chem. Soc. Chem. Commun. 1993, 341.

Also, most of the primary aralkylamines of formula (II) are known substances and are commercially available or can be easily prepared by a method described in a literature. Examples of aryl groups are phenyl, naphthyl and the like, and these groups may be substituted at any positions with any numbers of lower alkyl groups such as methyl, ethyl, isopropyl, butyl, isobutyl, sec-butyl and tert-butyl, or any numbers of lower alkoxy groups such as methoxy, ethoxy and isopropoxy. Preferable compounds of formula (II) are unsubstituted primary aralkylamines and most preferably benzylamine.

The compound of formula (I) and the compound of formula (II) may be used in a molar ratio of 1.2:1 to 1:5, and a preferable molar ratio is 1.1:1 to 1:1.5.

Hydrogen pressure may be in a range of 1 to 50 kg/cm², and preferably in a range of 2 to 30 kg/cm².

The solvent used may be any solvents so far as they do not prevent the progress of the reaction. Preferable solvents are alcohols, in particular, lower alcohols such as methanol, ethanol, propanol, 2-propanol and butanol. Most preferable solvents are methanol and ethanol. The solvent is used in an amount of 1.5 to 50 times (vol/wt), preferably in an amount of 5 to 40 times, per the compound of formula (I).

The catalyst for catalytic reduction may be any catalysts so far as they cause reductive amination. Preferable catalysts are platinum catalysts and Pt/C, PtO₂ and the like may be used. The catalyst maybe used in an amount of 1 to 30% (wt/wt), preferably in an amount of 5 to 20%, per the compound of formula (I) .

The reaction may be carried out within a temperature range of 0°C to 150°C, preferably 0°C to 50°C. Reaction time is 10 minutes to 10 hours, preferably 1 to 8 hours.

To the compound of formula (III) thus obtained, a suitable acid is added to prepare a crystalline salt, which may be recrystallized to improve both diastereomer purity (erythro/threo ratio) and optical purity [(1R,2S)/(1S,2R) ratio].

The acid used in this purpose may be any acids so far as they give crystalline salts suitable for such purification. Preferable acids are inorganic acids and most preferably hydrochloric acid.

The solvent used in recrystallization may be any solvents so far as they give a desired effect of purification. Preferable solvents are lower alcohols such as methanol, ethanol, propanol and 2-propanol, and more preferably 2-propanol.

The temperature of recrystallization is not specifically restricted so far as the desired purification is achieved, and may be in a range of -30°C to the boiling point of the solvent used, preferably in a range of 0°C to 80°C.

The salt purified by recrystallization may be treated with an aqueous alkali solution according to a conventional method to isolate a pure compound of formula (III) as a free amine.

The compound of formula (III) may be subjected to hydrogenolysis under hydrogen atmosphere in a suitable solvent in the presence of a catalyst for catalytic reduction to remove the N-aralkyl group and to obtain the compound of formula (IV).

Hydrogen pressure may be in a range of 1 to 50 kg/cm², and preferably in a range of 1 to 30 kg/cm².

The solvent used may be any solvents so far as they do not prevent the progress of the reaction. Preferable solvents are alcohols, in particular, lower alcohols such as methanol, ethanol, propanol, 2-propanol and butanol. Most preferable solvents are methanol and ethanol. The solvent is used in an amount of 1.5 to 50 times (vol/wt), preferably in an amount of 5 to 50 times, per the compound of formula (III).

The catalyst for catalytic reduction may be any catalysts so far as they cause hydrogenolysis. Preferable catalysts are palladium catalysts and Pd/C, Pd(OH)₂/C and the like may be used. The catalyst may be used in an amount of 0.5 to 40% (wt/wt), preferably in an amount of 5 to 30%, per the compound of formula (III).

The reaction may be carried out within a temperature range of 0°C to 150°C, preferably 0°C to 50°C. Reaction time is 10 minutes to 10 hours, preferably 1 to 8 hours.

The compound of formula (IV) thus obtained may be purified as a crystalline salt by the method described below, if necessary.

According to the present process, it is also possible to directly obtain the compound of formula (IV) by reacting the compound of formula (I) with the compound of formula (II) under hydrogen atmosphere in a suitable solvent in the presence of a suitable catalyst for catalytic reduction.

Hydrogen pressure in this case may be in a range of 1 to 50 kg/cm², preferably in a range of 2 to 30 kg/cm².

The solvent used may be any solvents so far as they do not prevent the progress of the reaction. Preferable solvents are alcohols, in particular, lower alcohols such as methanol, ethanol, propanol, 2-propanol and butanol. Most preferable solvents are methanol and ethanol. The solvent is used in an amount of 1.5 to 50 times (vol/wt), preferably in an amount of 5 to 20 times, per the compound of formula (I).

The catalyst for catalytic reduction in this case may be any catalysts so far as they can cause both reductive amination and hydrogenolysis. Preferable catalysts are palladium catalysts and Pd/C, Pd(OH)₂/C and the like may be used. The catalyst maybe used in an amount of 0.5 to 40% (wt/wt), preferably in an amount of 3 to 30%, per the compound of formula (I).

It is also possible to introduce the above catalyst for catalytic reduction by dividing when the reductive amination reaction and the hydrogenolysis reaction are carried out. By introducing the catalyst as such, the total amount of the catalyst used may be decreased as compared with the case where all the catalyst is introduced at once when the reductive amination reaction is carried out.

The reaction may be carried out within a temperature range of 0°C to 150°C, preferably 0°C to 60°C. Reaction time is 10 minutes to 10 hours, preferably 1 to 8 hours.

To the compound of formula (IV) thus obtained, a suitable acid is added to prepare a crystalline salt, which may be recrystallized to improve both diastereomer purity (erythro/threo ratio) and optical purity [(1R,2S)/(1S,2R) ratio].

The acid used in this purpose may be any acids so far as they give crystalline salts suitable for such purification. Preferable acids are inorganic acids and most preferably sulfuric acid in view of the effect of purification.

The solvent used in recrystallization may be any solvents so far as they give a desired effect of purification. A mixture of solvents may be used. Preferable solvents are lower alcohols such as methanol, ethanol, propanol and 2-propanol. More preferable solvents are a mixed solvent of a lower alcohol and water, and most preferable solvents are a mixed solvent of methanol and water. The mixing ratio is methanol:water = 20:1 to 1:1, preferbly methanol:water = 10:1 to 2:1 by weight ratio.

The temperature of recrystallization is not specifically restricted so far as the desired purification is achieved, and may be in a range of -20°C to the boiling point of the solvent used, preferably in a range of 0°C to 60°C.

The salt purified by recrystallization may be treated with an aqueous alkali solution according to a conventional method to isolate a pure compound of formula (IV) as a free amine.

### EXAMPLES

The present invention is illustrated in more detail by the following examples, but it is not limited thereto.

In these examples, the erythro/threo ratio was expressed by an area ratio in HPLC (high performance liquid chromatography) analysis or in GLC (gas-liquid chromatography) analysis. Also, the optical purity was expressed by an enantiomer excess percent (%ee).

### Example 1: L-erythro-(1R,2S)-2-(N-benzylamino)-1-phenylpropan-1-ol

A mixture of L- (R) -phenylacetylcarbinol (purity 79.1%; 9.50 g, 50.0 mmol), benzylamine (5.37 g, 50 mmol), 5% Pt/C (1.26 g) and ethanol (300 ml) was stirred for 8 hours at 20°C under hydrogen atmosphere (3 kg/cm²). The reaction mixture was filtered and the filtrate was concentrated under reduced pressure to obtain crude L-erythro-(1R,2S)-2-(N-benzylamino)-1-phenylpropan-1-ol (13.95 g).
Erythro:threo = 92.6:7.4.

| Conditions for HPLC analysis: | |
|---|---|
| Column | DAICEL CROWNPACK CR (+) (4.0 mmΦ x 150 mm); |
| Elute | MeOH:0.1N HClO₄ aq. = 15:85 (v/v); |
| Detection | UV 210 nm; |
| Flow rate | 0.8 ml/min.; |
| Column temperature | 40°C; |
| Measuring time | 70 min.; |
| t_{R} | erythro-2-(N-benzylamino)-1-phenylpropan-1-ol, 44.3 min.; threo-2(N-benzylamino)-1-phenylpropan-1-ol, 52.4 min. |

To the resultant crude L-erythro-(1R,2S)-2-(N-benzylamino)-1-phenylpropan-1-ol (8.0g), 10% hydrochloric acid/methanol (TOKYO CHEMICAL INDUSTRY CO., LTD.; 32.0 g) was added, and the mixture was concentrated under reduced pressure. 2-Propanol (32.0 g) was added to the residue and the mixture was heated to 70°C to dissolve the residue. Subsequently, the mixture was cooled to 10°C. The crystals precipitated were filtered and dried to obtain a hydrochloric acid salt of L-erythro-(1R,2S)-2-(N-benzylamino)-1-phenylpropan-1-ol (4.46 g). 2N Aqueous NaOH solution (16 ml) was added to the salt and the mixture was extracted with ether (20 ml). The ether extract was concentrated under reduced pressure to obtain free L-erythro-(1R,2S)-2-(N-benzylamino)-1-phenylpropan-1-ol (3.39 g).
¹H-NMR (400 MHz) δ(CDCl₃): 0.85 (3H, d, J=6.4 Hz), 1.90-2.60 (2H, m), 2.98 (1H, m), 3.87 (2H, s), 4.78 (1H, d, J=4.0 Hz), 7.15-7.40 (10H, m) ppm.
Purity: 98.63%.

| Conditions for HPLC analysis: | |
|---|---|
| Column | Nacalai Tesque COSMOSIL PACKED COLUMN 5C18-AR (4.6 mmΦ x 250 mm) ; |
| Elute | MeCN:H₂O:AcOH:SDS (sodium dodecyl sulfate) = 450 ml:550 ml:0.2 ml:1.2 g; |
| Detection | UV 210 nm; |
| Flow rate | 1.0 ml/min.; |
| Column temperature | room temperature; |
| Measuring time | 30 min.; |
| t_{R} | L-erythro-(1R,2S)-2-(N-benzylamino)-1-phenylpropan-1-ol, 19.54 min. |
| | Erythro : threo = 99.98:0.02. |

| Conditions for HPLC analysis: | |
|---|---|
| Column | DAICEL CROWNPACK CR (+) (4.0 mmΦ x 150 mm); |
| Elute | MeOH:0.1N HClO₄ aq. = 15:85 (v/v); |
| Detection | UV 210 nm; |
| Flow rate | 0.8 ml/min.; |
| Column temperature | 40°C; |
| Measuring time | 70 min.; |
| t_{R}: | erythro-2-(N-benzylamino)-1-phenylpropan-1-ol, 44.3 min.; threo-2-(N-benzylamino)-1-phenylpropan-1-ol, 52.4 min. |

### Example 2: L-erythro-(1R,2S)-2-amino-1-phenylpropan-1-ol

A mixture of L-erythro-(1R,2S)-2-(N-benzylamino)-1-phenylpropan-1-ol (3.00 g) obtained in Example 1, 20% Pd(OH)₂/C (0.62 g) and methanol (124 ml) was stirred for 7.3 hours at 24°C under hydrogen atmosphere (5 kg/cm²). The reaction mixture was filtered and the filtrate was concentrated under reduced pressure to obtain L-erythro-(1R,2S)-2-amino-1-phenylpropan-1-ol (1.83 g).
¹H-NMR (400 MHz) δ(CDCl₃) : 0.94 (3H, d, J=6.8 Hz), 1.90-2.60 (3H, m), 3.08 (1H, dd, J=4.8 Hz, 6.8 Hz), 4.44 (1H, d, J=4.8 Hz), 7.20-7.40 (10H, m) ppm.
Erythro:threo = 99.70:0.30.

| Conditions for GLC analysis: | |
|---|---|
| Column | SUPELCO α-DEX™ 120 (30 m x 0.025 mm ID; Film, 0.25 µm); |
| Column temperature | 100°C → 150°C (2.0°C/min., 25 min.) + 150°C (25 min.); |
| Injection temperature | 250°C; |
| Detection temperature | 300°C; |
| Carrier gas | He; |
| Flow rate | 5.0 ml/min.; |
| Split ratio | 1:100; |
| Detection | FID; |
| Measuring time | 45 min.; |
| t_{R} | threo-2-amino-1-phenylpropan-1-ol, 36.0 min.; erythro-2-amino-1-phenylpropan-1-ol, 37.6 min. |
| Optical purity | 97.1%ee. |

| Conditions for HPLC analysis: | |
|---|---|
| Column | DAICEL CROWNPACK CR (+) (4.0 mmΦ x 150 mm); |
| Elute | HClO₄ aq. (pH 2.0); |
| Detection | UV 210 nm; |
| Flow rate | 0.6 ml/min.; |
| Column temperature | room temperature; |
| Measuring time | 40 min.; |
| t_{R} | L-erythro-(1R,2S)-2-amino-1-phenylpropan-1-ol, 18.95 min.; |
| | D-erythro-(1S,2R)-2-amino-1-phenylpropan-1-ol, 23.36 min. |

### Example 3: L-erythro-(1R,2S)-2-amino-1-phenylpropan-1-ol

A mixture of L-(R) -phenylacetylcarbinol (purity 79.1%; 4.75 g, 25.0 mmol), benzylamine (2.68 g, 25 mmol), 20% Pd(OH)₂ (1.00 g) and methanol (47 ml) was stirred for 6 hours at 50°C under hydrogen atmosphere (5 kg/cm²). The reaction mixture was filtered and the residue was washed with methanol. The filtrate and the washed liquor were combined and concentrated under reduced pressure to obtain crude L-erythro-(1R,2S)-2-amino-1-phenylpropan-1-ol (5.67 g).
Erythro:threo = 88.3:11.7.

| Conditions for GLC analysis: | |
|---|---|
| Column | SUPELCO α-DEX™ 120 |
| | (30 m x 0.025 mm ID; Film, 0.25 µm); |
| Column temperature | 100°C → 150°C (2.0°C/min., 25 min.) |
| | + 150°C (25 min.); |
| Injection temperature | 250°C; |
| Detection temperature | 300°C; |
| Carrier gas | He; |
| Flow rate | 5.0 ml/min.; |
| Split ratio | 1:100; |
| Detection | FID; |
| Measuring time | 45 min.; |
| t_{R} | threo-2-amino-1-phenylpropan-1-ol, 36.0 min.; erythro-2-amino-1-phenylpropan-1-ol, 37.6 min. |
| Optical purity | 85.8%ee. |

| Conditions for HPLC analysis: | |
|---|---|
| Column | DAICEL CROWNPACK CR (+) (4.0 mmΦ x 150 mm); |
| Elute | HClO₄ aq. (pH 2.0); |
| Detection | UV 210 nm; |
| Flow rate | 0.6 ml/min.; |
| Column temperature | room temperature; |
| Measuring time | 40 min.; |
| t_{R} | L-erythro-(1R,2S)-2-amino-1-phenylpropan-1-ol, 18.95 min.; |
| | D-erythro-(1S,2R)-2-amino-1-phenylpropan-1-ol, 23.36 min. |

### Example 4: L-erythro-(1R,2S)-2-amino-1-phenylpropan-1-ol

A mixture of L-(R)-phenylacetylcarbinol (purity 68.3%; 65.95 g, 300 mmol), benzylamine (36.82 g, 343 mmol), recovered 5% Pd/C (3.60 g) and methanol (139 ml) was stirred for 4 hours at 5°C under hydrogen atmosphere (9 kg/cm²).

Subsequently, fresh 5% Pd/C (8.70 g, water content: 53.4%) was added to the mixture, the reaction temperature was raised to 60°C, and the mixture was stirred for 2 hours and one quarter. The reaction mixture was filtered and the residue was washed with methanol (20 ml). The filtrate and the washed liquor were combined to obtain a methanol solution (231.70 g) of L-erythro-(1R,2S)-2-amino-1-phenylpropan-1-ol.
Erythro:threo = 92.75:7.25 (conditions for GLC analysis are the same as in Example 3);
Optical purity: 85.1%ee (conditions for HPLC analysis are the same as in Example 3).

### Example 5: L-erythro-(1R,2S)-2-amino-1-phenylpropan-1-ol bisulfate

Crude L-erythro-(1R,2S)-2-amino-1-phenylpropan-1-ol (erythro:threo = 88.9:11.1; optical purity = 75.6%ee)(5.94 g) prepared in a similar manner as described in Example 3 was dissolved in methanol (10 g) and deionized water (4.0 g) and 97% sulfuric acid (1.896 g, 18.75 mmol) was added dropwise to the solution with stirring. The mixture was heated with stirring and the temperature was raised to a reflux temperature over 30 minutes . The homogenized mixture was refluxed for one hour and then cooled to 10°C over one hour. In the course of the cooling, the mixture was inoculated with a small amount of L-erythro-(1R,2S)-2-amino-1-phenylpropan-1-ol bisulfate when the internal temperature dropped to 50°C. The crystals precipitated were filtered, washed with methanol (3.0 g) and then dried to obtain L-erythro-(1R,2S)-2-amino-1-phenylpropan-1-ol bisulfate (1.83 g). A small amount of the product was treated by the method described in Example 1, and the erythro:threo ratio and the optical purity were measured for the resultant free L-erythro-(1R,2S)-2-amino-1-phenylpropan-1-ol.
Erythro:threo = 98.2:1.6.

| Conditions for GLC analysis: | |
|---|---|
| Column | SUPELCO α-DEX™ 120 (30 m x 0.025 mm ID; Film, 0.25 µm) ; |
| Column temperature | 100°C → 150°C (2.0°C/min., 25 min.) + 150°C (25 min.); |
| Injection temperature | 250°C; |
| Detection temperature | 300°C; |
| Carrier gas | He; |
| Flow rate | 5.0 ml/min.; |
| Split ratio | 1:100; |
| Detection | FID; |
| Measuring time | 45 min.; |
| t_{R} | threo-2-amino-1-phenylpropan-1-ol, 36.0 min.; erythro-2-amino-1-phenylpropan-1-ol, 37.6 min. |
| Optical purity | 98.6%ee. |

| Conditions for HPLC analysis: | |
|---|---|
| Column | DAICEL CROWNPACK CR (+) (4.0 mmΦ x 150 mm); |
| Elute | HClO₄ aq. (pH 2.0); |
| Detection | UV 210 nm; |
| Flow rate | 0.6 ml/min.; |
| Column temperature | room temperature; |
| Measuring time | 40 min.; |
| t_{R} | L-erythro-(1R,2S)-2-amino-1-phenylpropan-1-ol, 18.95 min.; |
| | D-erythro-(1S,2R)-2-amino-1-phenylpropan-1-ol, 23.36 min. |

### INDUSTRIAL UTILIZATION

The process according to the present invention is advantageous in respect of stereoselectivity as compared with prior processes and can efficiently convert L-(R)-phenylacetylcarbinol to L-erythro-(1R,2S)-2-amino-1-phenylpropan-1-ol.

## Claims

1. A process for preparing L-erythro-(1R,2S)-2-amino-1-phenylpropan-1-ol of formula (IV): 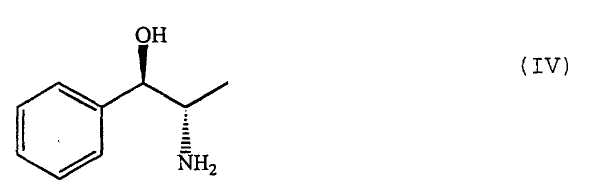 **characterized in that** L- (R)-phenylacetylcarbinol of formula (I): 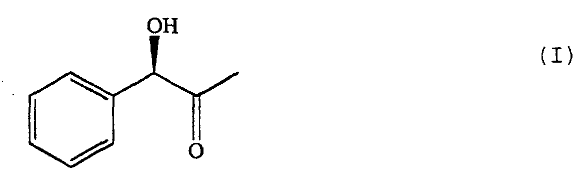 is reductively aminated, under catalytic reduction conditions, with a primary aralkylamine of formula (II):
ArCH₂NH₂ (II)
wherein Ar is an aryl group, and subsequently,
the resultant L-erythro-(1R,2S)-2-(N-aralkylamino)-1-phenylpropan-1-ol of formula (III): 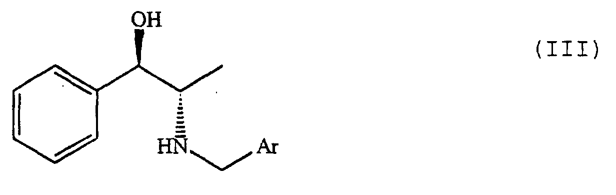 is catalytically reduced to remove the N-aralkyl group hydrogenolytically.

2. The process according to claim 1 wherein a platinum catalyst is used for reductive amination of the compound of formula (I) with the compound of formula (II) under catalytic reduction conditions, and the compound of formula (III) is isolated and purified and then subjected to catalytic reduction using a palladium catalyst, by which the N-aralkyl group is hydrogenolyzed to obtain the compound of formula (IV).

3. The process according to claim 2 further including a purification step by recrysallization of a crystalline salt obtained by adding a suitable acid to the compound of formula (III).

4. A process for preparing L-erythro-(1R,2S)-2-amino-1-phenylpropan-1-ol of formula (IV): 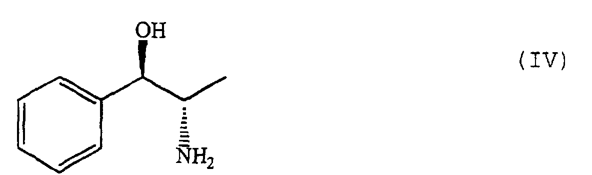 **characterized in that** L-(R)-phenylacetylcarbinol of formula (I): 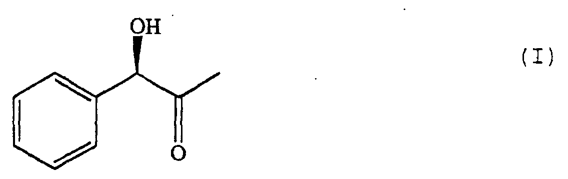 is reacted with a primary aralkylamine of formula (II):
ArCH₂NH₂ (II)
wherein Ar is an aryl group, in the presence of a catalyst for catalytic reduction which can cause both reductive amination and hydrogenolysis.

5. The process according to claim 3 or 4 further including a purification step by recrysallization of a crystalline salt obtained by adding a suitable acid to the compound of formula (IV).

6. The process according to any one of claims 1 to 5 wherein the compound of formula (II) is benzylamine.
